Europäisches Patentamt

European Patent Office

Office européen des brevets

(11) Publication number: **0 414 446 A2**

# EUROPEAN PATENT APPLICATION

(21) Application number: **90309003.3**

(22) Date of filing: **16.08.90**

(51) Int. Cl.⁵: **G01M 15/00**

(30) Priority: **19.08.89 GB 8918956**

(43) Date of publication of application:
**27.02.91 Bulletin 91/09**

(84) Designated Contracting States:
**AT BE CH DE ES FR GR IT LI LU NL SE**

(71) Applicant: **ENGINE TEST TECHNIQUE LIMITED**
**Lord Street**
**Lytham St.Annes, Lancashire FY8 3DF(GB)**

(72) Inventor: **Brown, Peter**
**213 Red Bank Road Bispham**
**Blackpool Lancashire FY2 9ET(GB)**

(74) Representative: **Attfield, Donald James et al**
**BROOKES, MARTIN & WILSON Prudential**
**Buildings 5 St. Philip's Place**
**Birmingham B3 2AF(GB)**

(54) **Gas analysis.**

(57) A method for determining the concentration of carbon monoxide, carbon dioxide and hydrocarbons in motor vehicle exhaust gases, comprises establishing a flow of the gas, filtering and directing the gas to a container (10) having an outlet to atmosphere, establishing a flow of a purging gas and alternately directing the purging gas and a portion of the exhaust gas withdrawn from the container (10) through a non-dispersive infra-red analysis cell (15,100-107) having a thin wall construction and wherein the gas is analysed by conventional methods, the cell being recalibrated during purging. The purge gas and exhaust gas are alternately directed through the cell (15, 100-107) by a solenoid valve (12) controlling the connection (14) of the cell to the purge gas or container (10) and the operation of a pump (11) located on the gas outlet side of the cell which draws the purge gas through the cell at a higher rate than the gas to be analysed.

The method provides for rapid warm-up of the cell and for reduced rate of contamination of the cell by the exhaust gases.

FIG 1

EP 0 414 446 A2

## GAS ANALYSIS

The present invention relates to a method and apparatus for gas analysis especially in relation to analysis of exhaust gas from internal combustion engines.

In order to measure the amount of particular gases in exhaust gas, infra-red optical benches have been adapted to detect the automotive gases of particular interest - namely carbon dioxide ($CO_2$), carbon monoxide (CO) and hydro carbons (HC). In aiming to provide an exhaust gas analyser which provides acceptable readings consideration has to be given to (i) warm up time, (ii) thermal stability, (without which there is usually an unacceptable drift in the readings which gives spurious results) and (iii) conditioning of the gas to remove such as excess water and particulate matter which if allowed to build up in the analyser cell would affect the readings.

Known optical benches have long warm up times which has led the trade to accepting delays as inevitable even of the order of half an hour. It is an aim of the present invention to provide a system which can be used within minutes of start-up.

In traditional analysis techniques, all of the gas being extracted from the exhaust pipe of a motor for analysis, is passed through the analysis cell irrespective of whether or not analysis is taking place. This is done to ensure that the gas in the cell is representative of the exhaust gas and clearly it is advantageous to maintain a flow of the gas being extracted for analysis. However, passing all of the gas continuously through the cell is disadvantageous since it gives rise to premature contamination of the cell.

It is known, for example from GBA-1530922, to alternately fill the cell with the gas to be analysed and with a reference or purging gas.

The present invention seeks to overcome certain of the disadvantages of known methods of gas analysis by providing a method of analysing gases to obtain the concentration of selected components, comprising establishing a flow of gas mixture to be analysed, establishing a flow of purging gas, alternately directing the gas mixture and purging gas through a non-dispersive infra-red gas analysis cell and processing the signals from the cell to indicate the concentration of the components, wherein a primary flow of gas mixture to be analysed is established and forwarded to a container zone from which zone a portion of said gas is directed through the said cell for analysis.

During the purging cycle the cell is recalibrated to take account of any change in operating conditions since the last purge operation, e.g. temperature fluctuation or deterioration of cell condition.

The purge and recalibration is done in a matter of seconds only so that gas measurement is only interrupted momentarily, typically for 3-5 seconds. The gas analysis time between purge operations is typically 5 minutes. The actual times are programmed into the apparatus so that cycling occurs automatically.

The cell construction is such that it is relatively stable for such analysis periods and so the readings are within permitted and desired accuracy. This is achieved in an advantageous manner by having the cell of relatively low mass providing warm up times of a matter of minutes.

From a further aspect, therefore, the invention provides an apparatus for use in determining the concentration of selected components present in a gas mixture comprising: a gas inlet, first means to establish a primary gas flow, a non-dispersive infra-red gas analysis cell, second means to alternately introduce said primary gas flow and a purging gas through the cell and means to process the signals from the said cell to calculate the concentration of selected components present in the gas mixture and to recalibrate the cell during purging, characterised in that said primary gas flow is directed to a container having gas inlet and exhaust ports and said second means acts to direct a portion of the gas from the said container through a second gas outlet port to the gas analysis cell.

The container may be a chamber having a first outlet for excess gases open to atmosphere and a second outlet for gases diverted to the analysis cell.

More particularly said second means includes a solenoid valve for controlling connection of the cell to the purge gas or to the gas container.

The apparatus further comprises a pump for alternately drawing the purging gas and the gas to be analysed through the analysis cell. The pump has differential operation rates whereby It exerts high suction in the purge mode to completely purge the cell of any gas to be analysed in 3-5 seconds, and a low suction setting which is operative to draw the gas to be analysed through the cell on switching of the solenoid. The pump and solenoid are interlinked and under the control of a microprocessor.

A separate pump 15 employed to extract gas to be analysed continuously from the gas source and direct it to a preliminary chamber from which a portion is taken for analysis. The gas is passed through a filter to extract water and water vapour and particulate matter prior to entering the preliminary chamber. A further pump extracts water from the water drain of the filter.

The apparatus includes microprocessors for controlling the cycling of the analysis cell, performing the recalibration, and for performing the gas analysis and communicating the appropriate data to a display. Warning means is included to signal onset of auto-zero/purge and during purging the last gas reading is held on the display.

In the construction of the infra-red gas analyser we provide a cell with a wall thickness in the range of 1 to 4mm thick, and preferably of the order of 2mm thick.

This is advantageous in allowing virtually instantaneous operation as the cell quickly stabilises with a drift characteristic which is acceptable.

By comparison we have found that thick walled cells of the order of 12mm can take several hours to stabilise during which time drift is unacceptably high to carry out reliable measurements.

We have found that the path length of the cell is preferably about 66.2mm and the bore diameter about 12mm. We also find it advantageous to have the cell constructed in parts which allows for disassembly on cleaning. This can be easily achieved by having a central section constructed as a thin walled tube with end flanges to which the source and detector housing are fitted by way of sealing rings. The detector housing accommodates the optics for detecting the gases to be measured and the source housing accommodates the infrared light source.

The present invention will now be described further, by way of example only, with reference to the accompanying drawings, in which:

Figure 1 is a schematic diagram of apparatus for use in the invention; and

Figure 2 is a perspective view of a preferred construction of analysis cell.

The present invention is concerned with the handling of gases to be analysed and to that end proposes a novel method and apparatus in relation thereto.

In performing gas analysis on gases such as exhaust gases from an internal combustion engine an optical gas analyser is employed in which energy of short wavelength (e.g. infra-red) is directed along an analysis chamber containing the gas to be analysed. The principle of operation is that the different constituent gases absorb infrared energy by different amounts and produce a shift in wavelength which is characteristic of each constituent. Accordingly collecting optics are arranged to respond only to a specific wavelength and can therefore select a particular gas from the total mix enabling precise measurements of relative percentages to be made. The priciples involved being well known are not therefore described further; they are not the subject of the present invention but are utilised in the method and apparatus in a manner which will be familiar to one skilled in the art.

Electronics associated with the analysis cell are of a standard nature, being provided to filter and amplify signals from the individual gas detectors in the cell and calculate the percentage of such gas, using a microprocessor, then output to a display with selection for each of the gases being measured. Further electronics (2nd microprocessor) are used to control the method of analysis as described further hereinafter.

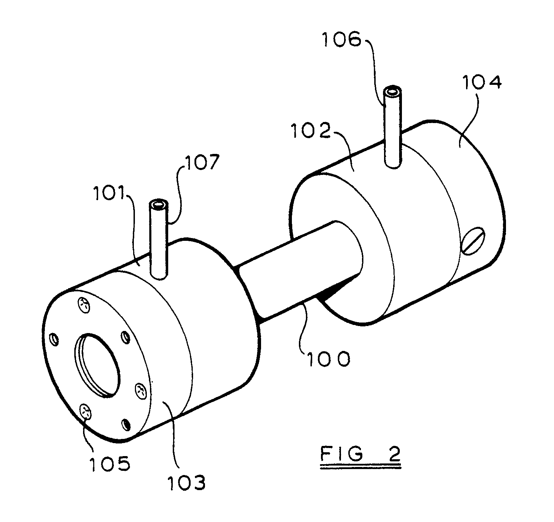

Referring firstly to Figure 2, an illustration of a preferred construction of optical bench for use in gas analysis apparatus of this invention. The bench comprises a cylindrical tube 100 33.3mm in length and having an internal diameter of the order of 12.6mm with a preferred wall thickness of 2mm.

Tube 100 is secured to flanges 101, 102 to provide a cell having a path length of 66.2mm. Detector housing 103 and source housing 104 are releasably secured to flanges 101, 102 respectively by screws 105. A gas inlet 106 is provided in flange 102 and a gas outlet 107 in flange 101. The details of the construction of the cell optics and of its operation are a matter of the art and are therefore not described herein.

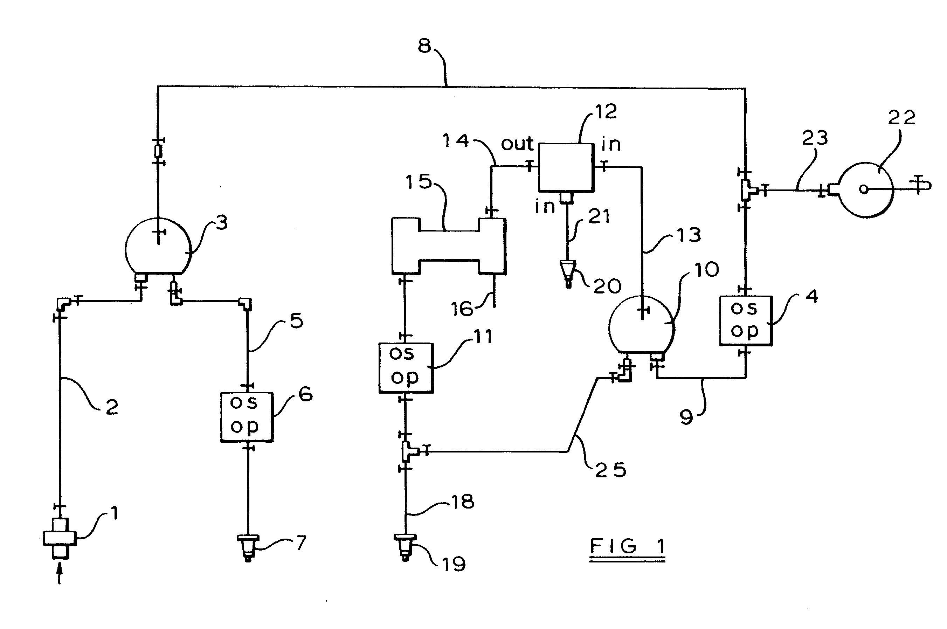

Reference is now made to Figure 1 in which the analysis cell is shown in outline at 15 with gas inlet line 14 and gas outlet line 17. Electrical connections to the cell are shown at line 16.

The gas to be analysed is extracted from its source (typically in automotive applications from the exhaust pipe of the engine) through gas inlet 1 under the action of pump 4 which draws the gas along line 2 through filter 3 along line 8. From pump 4 the gas is pushed along line 9 into chamber 10 which, in the present illustration, contains a second filter although such filter is not essential. Chamber 10 includes a gas outlet 25 through which excess gases leave the chamber. A pump 11, having differential operation rates, is located in outlet line 17 from cell 15 and draws a representative portion of gas from chamber 10 through sample line 13 and solenoid 12 to inlet line 14 of cell 15. Solenoid 12 also connects the cell with a source of purging gas (typically air) via inlet line 21.

Exhaust gases from pump 11 pass from line 18 to outlet 19 together with excess gases from chamber 10 via line 25. A pressure switch 22 is connected to line 8 via line 23 and acts to provide a visual or audible warning in the event of the occurence of a marked change in pressure occasioned, for example, by a blockage of filter 3. A pump 6 in outlet line 5 from filter 3 removes any water that may collect therein.

The apparatus operates as follows. On switch on pump 4 draws the gas mixture to be analysed from inlet 1 along lines 2, 8 and 9 and directs it into chamber 10. The gas being filtered by filter 3 and any residual water collecting in the filter being removed by pump 6 through outlet 7. With pump 11 operating at its lower rate solenoid valve 12 is switched to draw a portion of gas from container 10 through line 13 through the analysis cell 15 to exhaust via line 18 and outlet 19. This operation continues for approximately 5 minutes which time is sufficient for the apparatus to be warmed up whereupon a microprocessor (not shown) operates to switch the solenoid valve 12 to withdraw purged gas from inlet 20 at line 21 and pump 11 is simultaneously activated by the microprocessor to a second higher rate of operation to purge the sampling cell of residual gases. During purging signals from the cell 15 detectors are sampled and compared with the known reference or the purge gas and at a second microprocessor (not shown) memory resets at zero. This function is generally known as the "auto-zero" function and takes about 3 to 5 seconds. At the end of this period the apparatus is set for analysis and the solenoid valve 12 is switched by the first microprocessor to draw gas from chamber 10 and at the same time to restore pump 11 operation to its lower rate. The gas is thereby drawn through cell 15 and signals from the detectors in conjunction with the electronic means calculate the amount of the constituent gases which are present and the results displayed on a display (not shown) in any convenient manner. The analysis cycle continues for about 5 minutes.

At the end of the analysis period the apparatus automatically performs another "auto-zero" function as described above and then again reverts to gas analysis. An indication that auto-zero is about to commence is given by an audible warning which commences about 10 seconds before onset of auto-zero. During the auto-zero function the display holds the last reading.

In the system described above the first processor controls the auto-zero/purge system including the operation of the pumps and solenoid and a second microprocessor the linear circuit for display purposes.

**Claims**

1. A method of analysing gases to obtain the concentration of selected components, comprising establishing a flow of gas mixture to be analysed, establishing a flow of purging gas, alternately directing the gas mixture and purging gas through a non-dispersive infra-red gas analysis cell and processing the signals from the cell to indicate the concentration of the components, characterised in that a primary flow of gas mixture to be analysed is established and forwarded to a container zone from which zone a portion of said gas is directed through the said cell for analysis.

2. A method according to claim 1 or 2 wherein the primary flow of gas to be analysed is filtered before entering the said container zone.

3. A method according to claim 1, 2 or 3 wherein the purge gas is drawn through the cell at a greater rate than the gas to be analysed.

4. A method according to any one of the preceeding claims wherein the gas to be analysed is the exahust gas from an automobile and is analysed for carbon monoxide, carbon dioxide anbd hydrocarbons.

5. An apparatus for use in determining the concentration of selected components present in a gas mixture comprising; a gas inlet (1), first means (4) to establish a primary gas flow, a non-dispersive infra-red gas analysis cell (15, 100-107), second means (11, 12) to alternately introduce said primary gas flow and a purging gas through the cell (15, 100-107) and means to process sthe signals from the said cell to calculate the concentration of selected components present in the gas mixture, characterised in that said primary gas flow is directed to a container (10) having gas inlet (9) and exhaust ports (25) and said second means (11, 12) acts to direct a portion of the gas from the said container (10) through a second gas outlet port (13) to the gas analysis cell (15, 100-107).

6. An apparatus according to claim 5 wherein a first gas filter means (3) is provided in said primary gas flow and positioned before the said container (10).

7. An apparatus according to claim 5 or 6 wherein the said container (10) is a chamber having a first outlet port (25) for excess gases open to atmosphere and a second outlet port (13) for gases diverted to the analyser cell.

8. An apparatus according to claim 7 wherein the said container (10) includes second means to filter the gas to be analysed.

9. An apparatus according to any one of claims 5 to 8 wherein said second means (11, 12) to alternately direct gas mixture and purge gas into the analysis cell includes a solenoid valve (12) controlling the connection (14) of the said cell to the purging gas (21) or to the gas container (10).

10. An apparatus according to claim 9 wherein said second means (11, 12) includes a suction pump (11) positioned on the gas outlet side of said cell and having differential operation rates whereby the said pump exerts high suction during purging and low suction during gas analysis, the operation rate being controlled by signals from control means actuating the said solenoid (12).

11. An apparatus according to any one of claims 5-10 wherein a pressure switch (22) is provided between said first filter means (3) and said means (4) to establish a primary gas flow.

12. An apparatus according to any one of claims 5-11 wherein said cell (15, 100-107) has a wall thickness of 2mm, a length of 201.6mm, and a diameter of 12mm.

FIG 1

FIG 2